# EUROPEAN PATENT SPECIFICATION

(11) **EP 3 026 434 B1**
(45) Date of publication and mention of the grant of the patent: **05.01.2022**
(21) Application number: 14003958.7
(22) Date of filing: 25.11.2014
(51) Int. Cl.: G01N 33/68

(54) **A novel diagnostically relevant autoantibody**
Neuartiger, diagnostisch relevanter Autoantikörper
Nouvel autoanticorps d'intérêt pour diagnostics

(43) Date of publication of application: 01.06.2016
(73) Proprietor: Euroimmun Medizinische Labordiagnostika AG, 23560 Lübeck (DE)
(72) Inventor: Stöcker, Winfried, 23627 Gross Grönau (DE); Komorowski, Lars, 23909 Ratzeburg (DE); Miske, Ramona, 23552 Lübeck (DE); Scharf, Madeleine, 23564 Lübeck (DE); Denno, Yvonne, 23558 Lübeck (DE); Probst, Christian, 23909 Ratzeburg (DE)

(56) References cited:
- EP-A2- 0 897 982
- US-B1- 6 723 522
- VINCENT A ET AL: "Autoimmune channelopathies and related neurological disorders. (Review)", NEURON, CELL PRESS, US, vol. 52, no. 1, 5 October 2006 (2006-10-05), pages 123-138, XP002569453, ISSN: 0896-6273, DOI: 10.1016/J.NEURON.2006.09.024
- Kleopas A. Kleopa: "Autoimmune Channelopathies of the Nervous System", Current Neuropharmacology, 1 September 2011 (2011-09-01), pages 458-467, XP055185469, United Arab Emirates DOI: 10.2174/157015911796557966 Retrieved from the Internet: URL:http://www.eurekaselect.com/openurl/co ntent.php?genre=article&issn=1570159X&volu me=9&issue=3&spage=458 [retrieved on 2015-04-23]
- L. R. GAWENIS ET AL: "Colonic Anion Secretory Defects and Metabolic Acidosis in Mice Lacking the NBC1 Formula Cotransporter", JOURNAL OF BIOLOGICAL CHEMISTRY, vol. 282, no. 12, 19 January 2007 (2007-01-19), pages 9042-9052, XP055185439, ISSN: 0021-9258, DOI: 10.1074/jbc.M607041200
- MAJA NEIMAN ET AL: "Individual and stable autoantibody repertoires in healthy individuals", AUTOIMMUNITY, vol. 52, no. 1, 2 January 2019 (2019-01-02), pages 1-11, XP055701298, GB ISSN: 0891-6934, DOI: 10.1080/08916934.2019.1581774
- Susan Abu-Shakra ET AL: "Natural Hidden Antibodies", The Israel Medical Association Journal, vol. 9 1 September 2007 (2007-09-01), pages 748-749, XP055701299, DOI: 10.1046/j.1526-4610.2002.02199.x Retrieved from the Internet: URL:https://www.ima.org.il/FilesUploadPubl ic/IMAJ/0/46/23245.pdf

### SEQUENCE LISTING

<110> EUROIMMUN
<120> A novel diagnostically relevant autoantibody
<130> P00036
<160> 10
<170> BiSSAP 1.3
<210> 1
   <211> 468
   <212> PRT
   <213> Homo sapiens
<220>
   <223> NBC1 fragment (1-468)
<400> 1
   Met Glu Asp Glu Ala Val Leu Asp Arg Gly Ala Ser Phe Leu Lys His
   1 5 10 15
   Val Cys Asp Glu Glu Glu Val Glu Gly His His Thr Ile Tyr Ile Gly
   20 25 30
   Val His Val Pro Lys Ser Tyr Arg Arg Arg Arg Arg His Lys Arg Lys
   35 40 45
   Thr Gly His Lys Glu Lys Lys Glu Lys Glu Arg Ile Ser Glu Asn Tyr
   50 55 60
   Ser Asp Lys Ser Asp Ile Glu Asn Ala Asp Glu Ser Ser Ser Ser Ile
   65 70 75 80
   Leu Lys Pro Leu Ile Ser Pro Ala Ala Glu Arg Ile Arg Phe Ile Leu
   85 90 95
   Gly Glu Glu Asp Asp Ser Pro Ala Pro Pro Gln Leu Phe Thr Glu Leu
   100 105 110
   Asp Glu Leu Leu Ala Val Asp Gly Gln Glu Met Glu Trp Lys Glu Thr
   115 120 125
   Ala Arg Trp Ile Lys Phe Glu Glu Lys Val Glu Gln Gly Gly Glu Arg
   130 135 140
   Trp Ser Lys Pro His Val Ala Thr Leu Ser Leu His Ser Leu Phe Glu
   145 150 155 160
   Leu Arg Thr Cys Met Glu Lys Gly Ser Ile Met Leu Asp Arg Glu Ala
   165 170 175
   Ser Ser Leu Pro Gln Leu Val Glu Met Ile Val Asp His Gln Ile Glu
   180 185 190
   Thr Gly Leu Leu Lys Pro Glu Leu Lys Asp Lys Val Thr Tyr Thr Leu
   195 200 205
   Leu Arg Lys His Arg His Gln Thr Lys Lys Ser Asn Leu Arg Ser Leu
   210 215 220
   Ala Asp Ile Gly Lys Thr Val Ser Ser Ala Ser Arg Met Phe Thr Asn
   225 230 235 240
   Pro Asp Asn Gly Ser Pro Ala Met Thr His Arg Asn Leu Thr Ser Ser
   245 250 255
   Ser Leu Asn Asp Ile Ser Asp Lys Pro Glu Lys Asp Gln Leu Lys Asn
   260 265 270
   Lys Phe Met Lys Lys Leu Pro Arg Asp Ala Glu Ala Ser Asn Val Leu
   275 280 285
   Val Gly Glu Val Asp Phe Leu Asp Thr Pro Phe Ile Ala Phe Val Arg
   290 295 300
   Leu Gln Gln Ala Val Met Leu Gly Ala Leu Thr Glu Val Pro Val Pro
   305 310 315 320
   Thr Arg Phe Leu Phe Ile Leu Leu Gly Pro Lys Gly Lys Ala Lys Ser
   325 330 335
   Tyr His Glu Ile Gly Arg Ala Ile Ala Thr Leu Met Ser Asp Glu Val
   340 345 350
   Phe His Asp Ile Ala Tyr Lys Ala Lys Asp Arg His Asp Leu Ile Ala
   355 360 365
   Gly Ile Asp Glu Phe Leu Asp Glu Val Ile Val Leu Pro Pro Gly Glu
   370 375 380
   Trp Asp Pro Ala Ile Arg Ile Glu Pro Pro Lys Ser Leu Pro Ser Ser
   385 390 395 400
   Asp Lys Arg Lys Asn Met Tyr Ser Gly Gly Glu Asn Val Gln Met Asn
   405 410 415
   Gly Asp Thr Pro His Asp Gly Gly His Gly Gly Gly Gly His Gly Asp
   420 425 430
   Cys Glu Glu Leu Gln Arg Thr Gly Arg Phe Cys Gly Gly Leu Ile Lys
   435 440 445
   Asp Ile Lys Arg Lys Ala Pro Phe Phe Ala Ser Asp Phe Tyr Asp Ala
   450 455 460
   Leu Asn Ile Gln
   465
<210> 2
   <211> 16
   <212> PRT
   <213> Homo sapiens
<220>
   <223> NBC1 fragment2 (489-504)
<400> 2
   Gly Leu Leu Gly Asp Ala Thr Asp Asn Met Gln Gly Val Leu Glu Ser
   1               5                   10                  15
<210> 3
   <211> 28
   <212> PRT
   <213> Homo sapiens
<220>
   <223> NBC2 fragment3 (527-554)
<400> 3
   Ser Ser Thr Gly Pro Val Leu Val Phe Glu Arg Leu Leu Phe Asn Phe
   1               5                   10                  15
   Ser Lys Asp Asn Asn Phe Asp Tyr Leu Glu Phe Arg
               20                  25
<210> 4
   <211> 111
   <212> PRT
   <213> Homo sapiens
<220>
   <223> NBC1 fragment4 (581-691)
<400> 4
   Thr Arg Phe Thr Glu Glu Gly Phe Ser Ser Leu Ile Ser Phe Ile Phe
   1               5                   10                  15
   Ile Tyr Asp Ala Phe Lys Lys Met Ile Lys Leu Ala Asp Tyr Tyr Pro
               20                  25                  30
   Ile Asn Ser Asn Phe Lys Val Gly Tyr Asn Thr Leu Phe Ser Cys Thr
           35                  40                  45
   Cys Val Pro Pro Asp Pro Ala Asn Ile Ser Ile Ser Asn Asp Thr Thr
       50                  55                  60
   Leu Ala Pro Glu Tyr Leu Pro Thr Met Ser Ser Thr Asp Met Tyr His
   65                  70                  75                  80
   Asn Thr Thr Phe Asp Trp Ala Phe Leu Ser Lys Lys Glu Cys Ser Lys
                   85                  90                  95
   Tyr Gly Gly Asn Leu Val Gly Asn Asn Cys Asn Phe Val Pro Asp
               100                 105                 110
<210> 5
   <211> 15
   <212> **PRT**
   <213> Homo sapiens
<220>
   <223> NBC1 fragment5 (711-725)
<400> 5
   Lys Lys Phe Lys Thr Ser Pro Tyr Phe Pro Thr Thr Ala Arg Lys
   1               5                   10                  15
<210> 6
   <211> 29
   <212> PRT
   <213> Homo sapiens
<220>
   <223> NBC1 fragment6 (749-777)
<400> 6
   Asp Thr Pro Lys Leu Ile Val Pro Ser Glu Phe Lys Pro Thr Ser Pro
   1               5                   10                  15
   Asn Arg Gly Trp Phe Val Pro Pro Phe Gly Glu Asn Pro
               20                  25
<210> 7
   <211> 25
   <212> PRT
   <213> Homo sapiens
<220>
   <223> NBC1 framgent7 (798-822)
<400> 7
   Asp Gln Gln Ile Thr Ala Val Ile Val Asn Arg Lys Glu His Lys Leu
   1               5                   10                  15
   Lys Lys Gly Ala Gly Tyr His Leu Asp
               20                  25
<210> 8
   <211> 34
   <212> PRT
   <213> Homo sapiens
<220>
   <223> NBC1 fragment8 (848-881)
<400> 8
   Ser Ile Ala His Ile Asp Ser Leu Lys Met Glu Thr Glu Thr Ser Ala
   1               5                   10                  15
   Pro Gly Glu Gln Pro Lys Phe Leu Gly Val Arg Glu Gln Arg Val Thr
               20                  25                  30
   Gly Thr
<210> 9
   <211> 48
   <212> PRT
   <213> Homo sapiens
<220>
   <223> NBC1 fragment9 (902-949)
<400> 9
   Met Pro Val Leu Tyr Gly Val Phe Leu Tyr Met Gly Val Ala Ser Leu
   1               5                   10                  15
   Asn Gly Val Gln Phe Met Asp Arg Leu Lys Leu Leu Leu Met Pro Leu
               20                  25                  30
   Lys His Gln Pro Asp Phe Ile Tyr Leu Arg His Val Pro Leu Arg Arg
           35                  40                  45
<210> 10
   <211> 93
   <212> PRT
   <213> Homo sapiens
<220>
   <223> NBC1 fragment10 (987-1079)
<400> 10
   Arg Lys Gly Met Asp Tyr Leu Phe Ser Gln His Asp Leu Ser Phe Leu
   1               5                   10                  15
   Asp Asp Val Ile Pro Glu Lys Asp Lys Lys Lys Lys Glu Asp Glu Lys
               20                  25                  30
   Lys Lys Lys Lys Lys Lys Gly Ser Leu Asp Ser Asp Asn Asp Asp Ser
           35                  40                  45
   Asp Cys Pro Tyr Ser Glu Lys Val Pro Ser Ile Lys Ile Pro Met Asp
       50                  55                  60
   Ile Met Glu Gln Gln Pro Phe Leu Ser Asp Ser Lys Pro Ser Asp Arg
   65                  70                  75                  80
   Glu Arg Ser Pro Thr Phe Leu Glu Arg His Thr Ser Cys
                   85                  90

## Description

The present inventions relates to an isolated autoantibody, binding to a polypeptide comprising NBC1, wherein the autoantibody is in complex with said polypeptide, and a test kit for the diagnosis of a disease associated with neurological symptoms, comprising a polypeptide comprising NBC1 or a variant thereof having a sequence identity of at least 80 % to the sequence of NBC1 as represented by the UNIPROT accession number Q9Y6R1, wherein the variant has biological activity, wherein the test kit comprises, in addition, a means for detecting the complex comprising an autoantibody binding to NBC1, and wherein said means is a detectably labeled secondary antibody binding to the constant region of human IgG class antibodies.

Developing diagnostic systems for neurological diseases is a continuing challenge in biomedical science, not in the least because many symptoms encountered may be accounted for by a huge variety of causes including genetically-inherited diseases, drug abuse, malnutrition, infection, injury, psychiatric illness, immunological defects and cancer.

Since a neurological disease is rarely associated with a characteristic pattern of clinical symptoms, it is often difficult to provide a reliable diagnosis solely based on the observation and examination of the patients affected or their medical history.

The importance of an early diagnosis cannot be overemphasized. Many neurodegenerative disorders, most prominently Alzheimer's and Parkinson's diseases, cannot be cured, but drugs are available that may be used to slow down their progression. The earlier the diagnosis, the better the chances to exploit the spectrum of available drugs to the full benefit of the patient. In other cases, for example anti-NMDA receptor encephalitis, long-term damage may often be prevented by treating the patient at an early stage of the disease.

This holds all the more true in the case of neurological diseases associated with autoantibodies. In some cases, the link between a specific detectable autoantibody and a condition is sufficiently strong to allow for an immediate diagnosis based on the detection of the autoantibodies.

But even if it is not, the detection of autoantibodies may point the physician in charge to therapeutic means that may be used to ameliorate the patient's condition. There is a variety of widely used immunosuppressants that may be used to suppress the formation of pathogenic autoantibodies regardless of the nature of the autoantibody's target. Alternatively, apheresis may be used to remove autoantibodies from the patient's blood. In many cases, patients went on to lead a normal life following early diagnosis and treatment of a neurological autoimmune disease.

Diagnostic assays based on the detection of autoantibodies may also corroborate the diagnosis of diseases other than those associated with autoantibodies. If it turns out that a blood sample is devoid of specific autoantibodies, this result is likely to help the physician in charge exclude a range of possibilities and thus narrow down the spectrum of plausible conditions.

There are many examples of neurological conditions coinciding with the emergence of autoantibodies, including Neuromyelitis optica (Lennon, V. A., Wingerchuk, D. M., kryzer, T. J., Pittock, S. J., Lucchinetti, C. F., Fujihara, K., Nakashima, I., and Weinshenker, B. G. (2004) A serum autoantibody marker of neuromyelitis optica: distinction from multiple sclerosis, Lancet 364 (9451); 2106-2112), a disease characterized by loss of vision and spinal cord function, progressive encephalomyelitis (Hutchinson, M., Waters, P., McHugh, J., Gorman, G., O'Riordan, S., Connolly, S., Hager, H., Yu, P., Becker, C. M., and Vincent, A. (2008): Progressive encephalomyelitis, rigidity, and myoclonus: a novel glycine receptor antibody", Neurology, 71, 1291-2) and anti-NMDA receptor encephalitis (Dalmau, J., Gleichman, A. J., Hughes, E. G., Rossi, J. E., Peng, X., Lai, M., Dessain, S. K., Rosenfeld, M. R., Balice-Gordon, R., and Lynch, D. R. (2008), Anti-NMDA-receptor encephalitis: case series and analysis of the effects of antibodies, Lancet Neurology 7 (12), 1091-1098), which is associated with autonomic dysfunction, hypoventilation, cerebellar ataxia, hemiparesis, loss of consciousness, or catatonia. Whilst the role of autoantibodies and the nature of these conditions as such was previously poorly understood, many of these diseases can now be diagnosed and treated efficiently owing to the availability of assays based on the detection of autoantibodies. For example, a test based on the detection autoantibodies to NMDA receptor subunits (EP2483417; EP2649448) was widely appreciated by patients and medical doctors who, until then, were unaware of the autoimmune basis of the symptoms and consequently unable to provide adequate immunosuppressive treatment.

US 6723522 B1 discloses peptides and peptide fragments of NBC1. In addition, said document discloses monoclonal antibodies prepared in monkey, rabbit, dog, guinea pig, mouse, rat sheep and goat (cf. column 13, sixth paragraph) and polyclonal antibodies prepared in the before mentioned animals.

EP 0897982 A2 discloses pharmaceutical compositions comprising NBC1 and their use in therapy for specific diseases as ischaemia heart disease, arrhythmias, congestive heart disease, stroke and renal failure.

Therefore, it is paramount that new approaches to distinguish neurological conditions associated with autoantibodies from other conditions be developed.

Described herein and not part of the invention is an agent and a method for diagnosing a neurological disease, more specifically distinguishing diseases associated with neurological symptoms and the emergence of autoantibodies from other types of diseases.

Another problem underlying the present invention is to provide an autoantibody in complex with a polypeptide comprising NBC1 that, when found in a liquid sample taken from a patient, indicates that said patient is suffering from an autoimmune disease associated with neurological symptoms.

Described herein and not part of the invention is an agent and a method for diagnosing an autoimmune disease associated with neurological symptoms.

The problem underlying the present invention is solved by the subject-matter of the attached independent and dependent claims.

Described herein and not part of the invention is a method for diagnosing a disease, preferably a disease associated with neurological symptoms, comprising the step detecting in a sample from a patient an autoantibody binding to NBC1.

The sample, not part of the invention, may be a bodily fluid comprising antibodies, preferably selected from the group comprising whole-blood, serum, cerebrospinal fluid and saliva.

Also described herein and not part of the invention is an immobilized polypeptide comprising NBC1 or a variant thereof, preferably immobilized on a solid carrier.

Also described herein and not part of the invention is by a use of a polypeptide comprising NBC1 or a variant thereof for the diagnosis of a disease, preferably comprising the step detecting an autoantibody binding to NBC1.

Also described herein and not part of the invention is a polypeptide comprising NBC1 or a variant thereof, preferably immobilized, more preferably on a solid carrier, for use in the treatment of a disease.

The problem underlying the present invention is solved by an isolated autoantibody, binding to a polypeptide comprising NBC1, wherein the autoantibody is in complex with said polypeptide,

Also described herein and not part of the invention is a method for isolating an autoantibody binding to NBC1, comprising the steps
a) contacting a sample comprising the autoantibody with a polypeptide comprising NBC1 or a variant thereof under conditions compatible with formation of a complex, wherein said autoantibody binds to said polypeptide,
b) isolating the complex formed in step a),
c) dissociating the complex isolated in step b), and
d) separating the autoantibody from the polypeptide.

Also described herein and not part of the invention is a pharmaceutical composition comprising a polypeptide comprising NBC1 or a variant thereof, preferably for the treatment of a disease.

Also described herein and not part of the invention is a medical or diagnostic device comprising a polypeptide comprising NBC1 or a variant thereof.

Further, the problem underlying the present invention is solved by a test kit for the diagnosis of a disease associated with neurological symptoms, comprising a polypeptide comprising NBC1 or a variant thereof having a sequence identity of at least 80 % to the sequence of NBC1 as represented by the UNIPROT accession number Q9Y6R1, wherein the variant has biological activity,
wherein the test kit comprises, in addition, a means for detecting the complex comprising an autoantibody binding to NBC1, and wherein said means is a detectably labeled secondary antibody binding to the constant region of human IqG class antibodies.

The disease is associated with neurological symptoms,

Also described herein and not part of the invention is that the disease is a neurological disease, preferably selected from the group comprising Developmental structural disorders, Neurocutaneous disorders, Neurodegenerative dementias, Movement disorders, Spinocerebellar ataxias, Degenerative motor disorders, Non-degenerative neuromuscular disorders, Neurological infection disorders, Transmissible spongiform encephalopathy, Neurovascular disorders, Primary neurological tumors, Paraneoplastic syndromes, Demyelinating disorders of the central nervous system, Demyelinating disorders of peripheral nervous system, Autoimmune and inflammatory disorders, Traumatic disorders, Epilepsy, Sleep disorders and Headache, more preferably a Primary or Secondary headache, most preferably a Primary headache such as migraine.

Also described herein and not part of the invention is that the patient has or the disease is associated with one or more neurological symptoms comprising functional limb weakness, blackouts, sensory symptoms, pain, fatigue, sleep problems, poor memory and concentration, panic, dizziness, headache, low mood, facial spasms, visual problems, tremor, dystonia, walking problems, word finding difficulty, slurred speech, bladder symptoms, bowel symptoms, swallowing problems, pain, anxiety, jerks and twitches, autonomous instability,
preferably primary or secondary, preferably primary, headache, more preferably a headache selected from the group comprising cluster headaches, trigeminal neuralgia, hemicranias continua, familial headache, primary stabbing headache, primary cough headache, primary exertional headache and hypnic headache.

In a preferred embodiment of any aspect or embodiment of the invention, the polypeptide is provided in the form of a cell comprising a nucleic acid encoding said polypeptide or in the form of a tissue comprising said polypeptide.

In a preferred embodiment of any aspect or embodiment of the invention, the polypeptide is a recombinant and/or isolated polypeptide.

The present invention is based on the inventors' surprising finding that autoantibodies to NBC1 may be detected in samples from a number of patients suffering from neurological symptoms, but not in samples obtained from healthy subjects. The presence of such autoantibodies suggests that NBC1 activity and function are impaired in patients having NBC1 autoantibodies to the effect that a range of neurological symptoms occur,

The present invention relates to a polypeptide comprising a mammalian, the latter preferably selected from the group comprising rat (Q9JI66), mouse (088343), human (Q9Y6R1), bovine (Q9GL77), rabbit (Q9XSZ4) and pig (Q4U116) NBC1, more preferably human NBC1 or variants thereof, preferably immunogenic variants binding specifically to NBC1 autoantibodies. In a most preferred embodiment, the polypeptide comprises human NBC1 as represented by the UNIPROT accession number Q9Y6R1. Throughout this application, any data base code cited refers to the UNIPROT data base, more specifically the version accessible on-line on November 20, 2014. EMBL accession number AAC39840 represents a nucleotide sequence encoding human NBC1.

Human electrogenic sodium bicarbonate cotransporter 1 (NBC1), also referred to as NBCe1, hhNMC, HNBC1, KNBC, KNBC1, kNBC1, NBC, NBC2, NBCE1, pNBC, SLC4A4 or SLC4A5, is a membrane transport protein encoded by the *SLC4A4* gene.

NBC1 has a large N-terminal cytoplasmic region, a lipid-embedded transmembrane region and a C-terminal cytoplasmic tail. The N-terminal cytoplasmic region is tightly folded and predicted to form a domain structure, which is necessary for pH sensing and transport activity (Espiritu, D. J., Bernardo, A. A., Arruda, J. A., (2006) Role of NH2 and COOH termini in targeting, stability and activity of sodium bicarbonate cotransporter 1, Am. J. Physiol. Renal. Physiol. 2006; 291: F588-F596).

NBC1 is a 140-kDa glycoprotein containing 1035 amino acids composed of 14 transmembrane regions (TMs). A large extracellular loop containing two glycosylated sites is present between TMs 5 and 6. The oligomeric state of the cotransporter is dimeric and each monomeric subunit has independent transport activity. The hydrophilic C-terminus of NBC1 is involved in protein-protein interaction and targeting to the plasma membrane. It contains 15 consecutive, positively charged residues and the acidic motive LDSDNDD (reviewed in Kurtz, I., Zhu, Q. (2013) Structure, function, and regulation of the SLC4 NBCe1 transporter and its role in causing proximal renal tubular acidosis. Curr. Opin. Nephrol. Hypertens, 2013; 22:572-583.)

NBC1 mediates the coupled movement of sodium and bicarbonate ions across the plasma membrane of many cells, which is an electrogenic process with an apparent stoichiometry of 3 bicarbonate ions per sodium ion. Sodium bicarbonate is involved in bicarbonate secretion/absorption and regulation (reviewed in Soleimani, M., Burnham, C. E. (2000) Physiologic and molecular aspects of the Na+:HCO3- cotransporter in health and disease processes, Kidney Int. 2000; 57: 371-384).

NBC1 is the key transporter predominantly expressed in S1 and S2 proximal tubule cells in kidney that mediates basolateral Na⁺-base efflux thereby contributing to the reabsorption of 80% of the filtered bicarbonate load (Skelton, L. A., Boron, W. F., Zhou, Y.(2010) Acid-base transport by the renal proximal tubule, Journal of nephrology, 2010; 23 (Suppl 16):S4-18). By contrast, NBC1 works in the influx mode in the pancreatic duct cells (Shumaker, H., Amlal, H., Frizzell, R., Ulrich, C. D. II, Soleimani, M. (1999) CFTR drives Na+-HCO3- cotransport in pancreatic duct cells: A basis for defective HCO3 - secretion in CF, Am J Physiol. 1999; 276:C16-C25).

Interestingly, NBC1 dysfunction, more specifically lack of transport activity due to homozygous mutations in the SLC4A4 gene, is associated with familial migraine in a range of patients (Suzuki, M., Paesschen, W. V., Stalmans, I., Horita, S., Yamada, H., Bergmans, B. A., Legius, E., Riant, F., De Jonghe, P., Li, Y., Sekine, T., Igarashi, T., Fujimoto, I., Mikoshiba, K., Shimadzu, M., Skiohara, M., Braverman, N., Al-Gazali, L., Fujita, T., and Seki, G. (2010) Defective membrane expression of the Na+-HCO3- cotransporter NBCe1 is associated with familial migraine, PNAS 107 (26), 15963-15968).

The teachings of the present invention may not only be carried out using polypeptides, in particular a polypeptide comprising NBC1, or nucleic acids having the exact sequences referred to in this application explicitly, for example by function, name, sequence or accession number, or implicitly, but also using variants of such polypeptides or nucleic acids.

The term "variant", as used herein, may refer to at least one fragment of the full length sequence referred to, more specifically one or more amino acid or nucleic acid sequence which is, relative to the full-length sequence, truncated at one or both termini by one or more amino acids.

The term "variant" relates not only to at least one fragment, but also to a polypeptide or a fragment thereof comprising amino acid sequences that are at least 80, 85, 90, 92, 94, 95, 96, 97, 98 or 99 % identical to the reference amino acid sequence referred to or the fragment thereof, wherein amino acids other than those essential for the biological activity, for example the ability of an antigen to bind to an (auto)antibody, or the fold or structure of the polypeptide are deleted or substituted and/or one or more such essential amino acids are replaced in a conservative manner and/or amino acids are added such that the biological activity of the polypeptide is preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default settings.

In a preferred embodiment, variants may, in addition, comprise chemical modifications, for example isotopic labels or covalent modifications such as glycosylation, phosphorylation, acetylation, decarboxylation, citrullination, hydroxylation and the like. The person skilled in the art is familiar with methods to modify polypeptides. Any modification is designed such that it does not abolish the biological activity of the variant.

Moreover, variants may also be generated by fusion with other known polypeptides or variants thereof and comprise active portions or domains, preferably having a sequence identity of at least 80, 85, 90, 92, 94, 95, 96, 97, 98 or 99 % when aligned with the active portion of the reference sequence, wherein the term "active portion", as used herein, refers to an amino acid sequence, which is less than the full length amino acid sequence or, in the case of a nucleic acid sequence, codes for less than the full length amino acid sequence, respectively, and/or is a variant of the natural sequence, but retains at least some of the biological activity.

In a preferred embodiment, the term "variant" of a nucleic acid comprises nucleic acids the complementary strand of which hybridizes, preferably under stringent conditions, to the reference or wild type nucleic acid. Stringency of hybridization reactions is readily determinable by one of ordinary skilled in the art, and in general is an empirical calculation dependent on probe length, washing temperature and salt concentration. In general longer probes require higher temperatures for proper annealing, while shorter probes less so. Hybridization generally depends on the ability of denatured DNA to reanneal to complementary strands present in an environment below their melting temperature: The higher the degree of desired homology between the probe and hybridizable sequence, the higher the relative temperature which may be used. As a result, higher relative temperatures would tend to make the reaction conditions more stringent, while lower temperatures less so. For additional details and explanation of stringency of hybridization reactions, see Ausubel, F. M. (1995), Current Protocols in Molecular Biology. John Wiley & Sons, Inc. Moreover, the person skilled in the art may follow the instructions given in the manual Boehringer Mannheim GmbH (1993) The DIG System Users Guide for Filter Hybridization, Boehringer Mannheim GmbH, Mannheim, Germany and in Liebl, W., Ehrmann, M., Ludwig, W., and Schleifer, K. H. (1991) International Journal of Systematic Bacteriology 41: 255-260 on how to identify DNA sequences by means of hybridization. In a preferred embodiment, stringent conditions are applied for any hybridization, i.e. hybridization occurs only if the probe is 70 % or more identical to the target sequence. Probes having a lower degree of identity with respect to the target sequence may hybridize, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C - 68°C, approximately 56°C - 68°C, approximately 58°C - 68°C, approximately 60°C - 68°C, approximately 62°C - 68°C, approximately 64°C -68°C, approximately 66°C - 68°C. In a particularly preferred embodiment, the temperature is approximately 64°C - 68°C or approximately 66°C - 68°C. It is possible to adjust the concentration of salt to 0.2 x SSC or even 0.1 x SSC. Nucleic acid sequences having a degree of identity with respect to the reference or wild type sequence of at least 70, 80, 90, 91, 92, 93, 94, 95, 96, 97, 98, 99 % may be isolated. In a preferred embodiment, the term variant of a nucleic acid sequence, as used herein, refers to any nucleic acid sequence that encodes the same amino acid sequence and variants thereof as the reference nucleic acid sequence, in line with the degeneracy of the genetic code.

The variant of the polypeptide has biological activity. In a preferred embodiment, such biological activity is the ability to bind specifically to the NBC1 autoantibodies found in patients.

The polypeptide, which comprises NBC1 or a variant thereof, when used to carry out the teachings of the present invention, may be provided in any form and at any degree of purification, from tissues or cells comprising said polypeptide in an endogenous form, more preferably cells overexpressing the polypeptide, crude or enriched lysates of such cells, to purified and/or isolated polypeptide which is essentially pure. In a preferred embodiment, the polypeptide is a native polypeptide, wherein the term "native polypeptide", as used herein, refers to a folded polypeptide, more preferably to a folded polypeptide purified from tissues or cells, more preferably from mammalian cells or tissues, optionally from non-recombinant tissues or cells. In another preferred embodiment, the polypeptide is a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", "Purifying Challenging Proteins", "Recombinant Protein Purification", "Affinity Chromatography", "Ion Exchange Chromatography", "Gel Filtration (Size Exclusion Chromatography)", "Hydrophobic Interaction Chromatography", "Multimodal Chromatography" (2009/2010), published by GE Healthcare Life Sciences, and in Burgess, R. R., Deutscher, M. P. (2009), Guide to Protein Purification). In a preferred embodiment, a polypeptide is pure if at least 60, 70, 80, 90, 95 or 99 percent of the polypeptide in the respective sample consists of said polypeptide as judged by SDS polyacrylamide gel electrophoresis followed by Coomassie blue staining and visual inspection.

If the polypeptide comprising NBC1 or a variant thereof is provided in the form of tissue, it is preferred that the tissue is mammalian tissue, for example human, rat, primate, donkey, mouse, goat, horse, sheep, pig or cow, more preferably brain tissue, most preferably cerebellar tissue. If a cell lysate is used, it is preferred that the cell lysate comprises the membranes associated with the surface of the cell. If said polypeptide is provided in the form of a recombinant cell, it is preferred that the recombinant cell is a eukaryotic cell such as a yeast cell, more preferably a cell from a multicellular eukaryote such as a plant, mammal, frog or insect, most preferably from a mammal, for example rat, human, primate, donkey, mouse, goat, horse, sheep, pig or cow. For example, the cell may be a COS1, COS7, CHO, HL60, HEp-2, HeLa or HEK293 cell transfected with a nucleic acid functionally encoding the polypeptide. The person skilled in the art is familiar with methods for preparing, transfecting and culturing such cells, for example those described in Phelan, M. C. (2001), Basic Techniques in Mammalian Cell Tissue Culture, John Wiley.

The polypeptide used to carry out the inventive teachings, including any variants, is preferably designed such that it comprises epitopes recognized by and/or binds specifically to autoantibodies binding to NBC1. The person skilled in the art is familiar with guidelines used to design peptides having sufficient immunogenicity, for example those described in Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173. Briefly, it is desirable that the peptide meets as many as possible of the following requirements: (a) it has a high degree of hydrophilicity, (b) it comprises one or more residues selected from the group comprising aspartate, proline, tyrosine and phenylalanine, (c) is has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71.

The polypeptide, which comprises NBC1 or a variant thereof, when used according to the present invention, may be provided in any kind of conformation. For example, the polypeptide may be an essentially unfolded, a partially or a fully folded polypeptide. In a preferred embodiment, the polypeptide is folded in the sense that the epitopes that are essential for the binding to the inventive autoantibody, or the protein or variant thereof in its entirety, adopt the fold adopted by the native protein in its natural environment. The person skilled in the art is familiar with methods suitable to determine whether or not a polypeptide is folded and if it is, which structure it has, for example limited proteolysis, NMR spectroscopy, CD spectroscopy or X-ray crystallography (see for example Banaszak L. J. (2008), Foundations of Structural Biology, Academics Press, or Teng Q. (2013), Structural Biology: Practical Applications, Springer), preferably multidimensional NMR spectroscopy is used.

The polypeptide may be a fusion protein which comprises amino acid sequences other than those taken from NBC1, in particular a C-terminal or N-terminal tag, preferably a C-terminal tag, which is, in a preferred embodiment, as used herein, an additional sequence motif or polypeptide having a function that has some biological or physical function and may, for example, be used to purify, immobilize, precipitate or identify the polypeptide. In a more preferred embodiment, the tag is a sequence or domain capable of binding specifically to a ligand, for example a tag selected from the group comprising His tags, thioredoxin, maltose binding protein, glutathione-S-transferase, a fluorescence tag, for example from the group comprising green fluorescent protein.

The polypeptide may be an immobilized polypeptide. In a preferred embodiment, the term "immobilized", as used herein, refers to a molecule bound to a solid carrier insoluble in an aqueous solution, more preferably via a covalent bond, electrostatic interactions, encapsulation or entrapment, for example by denaturing a globular polypeptide in a gel, or via hydrophobic interactions, most preferably via one or more covalent bonds. Various suitable carriers, for example paper, polystyrene, metal, silicon or glass surfaces, microfluidic channels, membranes, beads such as magnetic beads, column chromatography media, biochips, polyacrylamide gels and the like have been described in the literature, for example in Kim, D., and Herr, A. E. (2013), Protein immobilizsation techniques for microfluidic assays, Biomicrofluidics 7(4), 041501. This way, the immobilized molecule, together with the insoluble carrier, may be separated from an aqueous solution in a straightforward manner, for example by filtration, centrifugation or decanting. An immobilized molecule may be immobilized in a reversible or irreversible manner. For example, the immobilization is reversible if the molecule interacts with the carrier via ionic interactions that can be masked by addition of a high concentration of salt or if the molecule is bound via a cleavable covalent bond such as a disulphide bridge which may be cleaved by addition of thiol-containing reagents. By contrast, the immobilization is irreversible if the molecule is tethered to the carrier via a covalent bond that cannot be cleaved in aqueous solution, for example a bond formed by reaction of an epoxide group and an amine group as frequently used to couple lysine side chains to affinity columns. The protein may be indirectly immobilized, for example by immobilizing an antibody or other entity having affinity to the molecule, followed by formation of a complex to the effect that the molecule-antibody complex is immobilized. Various ways to immobilize molecules are described in the literature, for example in Kim, D., Herr, and A. E. (2013), Protein immobilizsation techniques for microfluidic assays, Biomicrofluidics 7(4), 041501. In addition, various reagents and kits for immobilization reactions are commercially available, for example from Pierce Biotechnology.

It is essential that the sample used for the diagnosis in line with the present invention comprises antibodies, also referred to as immunglobulins. Typically the sample of a bodily fluid comprises a representative set of the entirety of the subject's immunglobulins. However, the sample, once provided, may be subjected to further processing which may include fractionation, centrifugation, enriching or isolating the entirety of immunglobulins or any immunglobulin class of the subject, which may affect the relative distribution of immunglobulins of the various classes.

The reagents, devices, methods and uses described throughout this application may be used for the diagnosis of a disease. The disease may be a neurological disease. The term "neurological disease", as used herein, refers to any disease associated with a defect of the nervous system, more preferably the central nervous system, more preferably the brain. The neurological disease may be selected from the group of neurological diseases associated with developmental structural disorders. The disease may be a psychiatric disease. The term "psychiatric disease", as used herein, is a subgroup of neurological diseases characterized in that it comprises a mind or behavioral pattern or anomaly that causes either suffering or an impaired ability to function in ordinary life.

Also described herein and not part of the invention is that the neurological disease is selected from the group comprising Developmental structural disorders, Neurocutaneous disorders, Neurodegenerative dementias, Movement disorders, Spinocerebellar ataxias, Degenerative motor disorders, Non-degenerative neuromuscular disorders, Neurological infection disorders, Transmissible spongiform encephalopathy, Neurovascular disorders, Primary neurological tumors, Neurological paraneoplastic syndromes, Demyelinating disorders of the central nervous system, Demyelinating disorders of peripheral nervous system, Autoimmune and Inflammatory disorders, Traumatic disorders, Epilepsy, Sleep disorders and Headaches.

Also described herein and not part of the invention is that the neurological disease is a developmental structural disorder selected from the group comprising Aicardi syndrome, Amniotic band syndrome, Anencephaly, Angelman syndrome, Aposthia, Arnold-Chiari malformation, Bannayan-Zonana syndrome, Bardet-Biedl syndrome, Barth syndrome, Basal cell nevus syndrome, Beckwith-Wiedemann syndrome, Benjamin syndrome, Caudal regression syndrome, Sotos syndrome cerebral gigantism, Cleidocranial dysostosis, Congenital central hypoventilation syndrome, Congenital diaphragmatic hernia, Congenital insensitivity to pain with anhidrosis, Costello syndrome, De lange syndrome, Encephalocele, Goldenhar syndrome, Holoprosencephaly, Huntington's disease, Hirschsprung's disease (or Congenital aganglionic megacolon), Incontinentia pigmenti, Intestinal neuronal dysplasia, Jacobsen syndrome, Katz syndrome, Laurence-moon syndrome, Lissencephaly, Marfan syndrome, Microcephaly, Lambert-eaton myasthenic syndrome, Myelokathexis, Neonatal jaundice, Neurofibromatosis, Neuronal ceroid-lipofuscinosis, Noonan syndrome, Nystagmus, Oculocerebrorenal syndrome, Rett syndrome, Robinow syndrome, Saethre-Chotzen syndrome, Schizencephaly, Smith-lemli-opitz syndrome, Spina bifida, Sturge-weber syndrome, Trichothiodystrophy, Waardenburg syndrome, Werner syndrome and Wolf-Hirschhorn syndrome.

Also described herein and not part of the invention is that the neurological disease is a neurocutaneous disorder selected from the group comprising Neurofibromatosis, Ataxia telangiectasia, Sturge-Weber syndrome, Von hippel-lindau disease, Incontinentia pigmenti, Nevoid basal cell carcinoma syndrome and Wyburn-Mason syndrome (Bonnet-Dechaume-Blanc syndrome).

Also described herein and not part of the invention is that the neurological disease is a neurodegenerative dementia selected from the group comprising Alzheimer's disease, Vascular dementia, Dementia with Lewy bodies, Mixed dementia, Parkinson's disease, Frontotemporal dementia, Creutzfedt-Jokob disease, Normal pressure hydrocephalus, Huntington's disease and Wernicke-Korsakoff syndrome.

Also described herein and not part of the invention is that the neurological disease is a movement disorder selected from the group comprising Akathisia, Akinesia, Homolateral synkinesis, Athetosis, Ataxia, Ballismus, Bradykinesia, Cerebral palsy, Chorea, Dyskinesia, Dystonia, Geniospasm, Metabolic general unwellness movement syndrome, Mirror movement disorder, Parkinson's disease, paroxysmal, Kinesigenic dyskinesia, Restless legs syndrome (Wittmaack-Ekboms disease), Stereotypic movement disorder, Stereotypy, Tourette's syndrome and Wilson's disease.

Also described herein and not part of the invention is that the neurological disease is a degenerative motor disorder selected from the group comprising Amyotrophic lateral sclerosis, Progressive bulbar palsy, Progressive muscular atrophy and Primary lateral sclerosis.

Also described herein and not part of the invention is that the neurological disease is a non-degenerative neuromuscular disorder selected from the group comprising Myasthenia gravis, Lambert-Eaton syndrome and Polymyositis.

Also described herein and not part of the invention is that the neurological disease is a neurological infection disorder selected from the group comprising Herpes simplex encephalitis, West nile virus neurologic infections, Creutzfeldt-Jakob disease, Neurosarcoidosis, Progressive multifocal leukoencephalopathy, AIDS-related viral infections of the nervous system and Chronic meningitis.

Also described herein and not part of the invention is that the neurological disease is a transmissible spongiforme encephalopathy selected from the group comprising Scrapie, Transmissible mink encephalopathy, Chronic wasting disease, Bovine spongiform encephalopathy, Feline spongiform encephalopathy, Exotic ungulate encephalopathy, Kuru, Creutzfeldt-Jakob disease, (New) variant Creutzfeldt-Jakob disease (vCJD, nvCJD), Gerstmann-Straeussler-Scheinker syndrome and Fatal familial insomnia.

Also described herein and not part of the invention is that the neurological disease is a neurovascular disorder selected from the group comprising Aneurysm, Dural arteriovenous fistula, Arteriovenous malformation (AVM, angioma), Cavernoma, Cerebral hemorrhage and Stroke.

Also described herein and not part of the invention is that the neurological disease is a primary neurological tumor selected from the group comprising Gliomas (Astrocytoma, Oligodendroglioma, Ependymoma), Meningiomas, Haemangioblastoma, Acoustic neuromas, Craniopharyngioma, Lymphomas, Germ cell tumor, Pineal region tumor, Primitive neuroectodermal tumor (including medulloblastoma), Haemangiopericytomas and Spinal cord tumor.

Also described herein and not part of the invention is that the neurological disease is a paraneoplastic syndrome selected from the group comprising Cushing syndrome, Hypercalcemia, Hyponatremia, Polycythemia, Trousseau syndrome, Hypoglycemia and Carcinoid syndrome.

Also described herein and not part of the invention is that the neurological disease is a demyelinating disorders of the central nervous system selected from the group comprising Multiple sclerosis, CNS Neuropathy such as those produced by Vitamin B12 deficiency, Central pontine myelinolysis, Myelopathy such as Tabes dorsalis (syphilitic Myelopathy), Leukoencephalopathy such as progressive multifocal, Leukoencephalopathy and Leukodystrophy.

Also described herein and not part of the invention is that the neurological disease is a demyelinating disorders of peripheral nervous system selected from the group comprising Guillain-Barré syndrome, Anti-MAG peripheral neuropathy, Charcot-Marie-Tooth Disease, Copper deficiency and Progressive inflammatory neuropathy.

Also described herein and not part of the invention is that the neurological disease is autoimmune or inflammatory disorder selected from the group comprising Rheumatoid arthritis, Systemic lupus erythematosus, Multiple sclerosis, Celiac sprue disease, Pernicious anemia, Vitiligo, Scleroderma, Psoriasis, Inflammatory bowel disease, Hashimoto's disease, Addison's disease, Graves' disease, Reactive arthritis, Sjögren's syndrome and Type 1 diabetes.

Also described herein and not part of the invention is that the neurological disease is a traumatic disorder selected from the group comprising Acute stress disorder, Adjustment disorder, Depersonalization disorder, Dissociative identity disorder, Panic disorder, Mental retardation, Generalized anxiety disorder and Post-traumatic stress disorder.

Also described herein and not part of the invention is that the neurological disease is epilepsy selected from the group comprising Idiopathic epilepsy, Symptomatic or Cryptogenic epilepsy, photosensitive epilepsy, Benign rolandic epilepsy, Lennox-Gastaut syndrome, Juvenile myoclonic epilepsy, Abdominal epilepsy, Absence seizures and Temporal lobe seizures.

Also described herein and not part of the invention is that the neurological disease is a sleep disorder selected from the group comprising Insomnia, Hypersomnia, Nocturnal enuresis, Bruxism, Obstructive sleep apnea, Delayed sleep phase syndrome, Advanced sleep phase syndrome, Non-24-hour sleep-wake syndrome, Hypopnea syndrome, Narcolepsy, Nocturia, Cataplexy, Night terror, Parasomnias, Restless leg syndrome, Periodic limb movement disorder, Rapid eye movement behavior disorder, Situational circadian rhythm sleep disorders, Sleep paralysis, Sleep walking, Snoring, Sleeping sickness and Sleeping talking and Sudden infant death syndrome.

Also described herein and not part of the invention is that the neurological disease is a headache selected from the group comprising primary headache and secondary headache.

Also described herein and not part of the invention is that the neurological disease is a Primary headache, wherein the primary headache is preferably selected from the group comprising Migraine, Tension-type headache, Trigeminal autonomic cephalalgias and other primary headache disorders.

Also described herein and not part of the invention is that the primary headache is a Migraine selected from the group comprising Typical aura with headache, Typical aura without headache, Familial hemiplegic migraine type 1, Familial hemiplegic migraine type 2, Familial hemiplegic type 3, Familial hemiplegic migraine other loci, Sporadic Hemiplegic migraine, Retinal migraine, Status migrainosus, Persistent aura without infarction, Migrainous infarction, Migraine aura-triggered seizure, Probable migraine with aura, Probable migraine without aura, Cyclical vomiting syndrome, Abdominal migraine, Benign paroxysmal vertigo and Benign paroxysmal toriticollis.

Also described herein and not part of the invention is that the primary headache is a Tension-type headache selected from the group comprising Infrequent episodic tension headache associated with pericranial tenderness, Infrequent episodic tension headache not associated with pericranial tenderness, Frequent episodic tension-type headache associated with pericranial tenderness, Frequent episodic tension-type headache not associated with pericranial tenderness, Chronic tension-type headache associated with pericranial tenderness, Chronic tension-type headache not associated with pericranial tenderness, Probable infrequent episodic tension-type headache, Probable frequent episodic tension-type headache and Probable chronic tension-type headache.

Also described herein and not part of the invention is that the primary headache is a Trigeminal automatic cephalalgia selected from the group comprising Episodic cluster headache, Chronic cluster headache, Episodic paroxysmal hemicranias, Chronic paroxysmal hemicranias, Episodic short-lasting unilateral neuralgiform headache attacks with conjunctival injection and tearing (SUNCT), Chronic SUNCT, Episodic short-lasting unilateral neuralgiform headache attacks with cranial autonomic symptoms (SUNA), Chronic SUNA, hemicrania continua, Probable cluster headache, Probable paroxysmal hemicrania, Probable short-lasting unilateral neuralgiform headache attacks and Probable hemicrania continua.

Also described herein and not part of the invention is that the primary headache is another primary headache disorder selected from the group comprising Probable cough headache, Probable primary exercise headache, Probable primary headache associated with sexual activity, Primary thunderclap headache, Headache attributed to external application of a cold stimulus, Headache attributed to ingestion or inhalation of a cold stimulus, Probable cold-stimulus headache, External-compression headache, External-traction headache, Probable external-pressure headache, Probable primary stabbing headache, Probable nummular headache, Probable hypnic headache and Probable new daily persistent headache.

Also described herein and not part of the invention is that the neurological disease is a Secondary headache, wherein the secondary headache is preferably selected from the group comprising Headache attributed to trauma or injury to the head and/or neck, Headache attributed to cranial, Headache attributed to cervical vascular disorder, Headache attributed to non-vascular intracranial disorder, Headache attributed to a substance or its withdrawal, Headache attributed to infection, Headache attributed to disorder of homoeostasis, Headache or facial pain attributed to disorder of the cranium, neck, eyes, ears, nose, sinuses, teeth, mouth or other facial or cervical structure, Headache attributed to psychiatric disorder, Painful cranial neuropathy and other facial pains.

Also described herein and not part of the invention is that the secondary headache is a headache attributed to trauma or injury to the head selected from the group comprising Acute headache attributed to moderate or severe traumatic injury to the head, Acute headache attributed to mild traumatic injury to the head, Persistent headache attributed to moderate or severe traumatic injury to the head, Persistent headache attributed to mild traumatic injury to the head, Acute headache attributed to whiplash, Persistent headache attributed to whiplash, Acute headache attributed to craniotomy and Persistent headache attributed to craniotomy.

Also described herein and not part of the invention is that the Secondary headache is a headache attributed to cranial or cervical vascular disorder selected from the group comprising Headache attributed to ischaemic stroke or transient ischaemic attack, Headache attributed to non-traumatic intracranial haemorrhage, Headache attributed to unruptured vascular malformation, Headache attributed to giant cell arteritis, Headache attributed to primary angiitis of the central nervous system, Headache attributed to secondary angiitis of the central nervous system, Headache or facial or neck pain attributed to cervical carotid or vertebral artery dissection, Post-endarterectomy headache, Headache attributed to carotid or vertebral angioplasty, Headache attributed to cerebral venous thrombosis, Headache attributed to an intracranial endovascular procedure, Angiography headache, Headache attributed to reversible cerebral vasoconstriction syndrome, Headache attributed to intracranial arterial dissection, Cerebral autosomal dominant arteriopathy with subcortical infarcts and leukoencephalopathy, Mitochondrial encephalopathy, Lactic acidosis, Stroke-like episodes, Headache attributed to another genetic vasculopathy and Headache attributed to pituitary apoplexy.

Also described herein and not part of the invention is that the Secondary headache is a headache attributed to non-vascular intracranial disorder selected from the group comprising Headache attributed to idiopathic intracranial hypertension, Headache attributed to intracranial hypertension secondary to metabolic, toxic or hormonal causes, Headache attributed to intracranial hypertension secondary to hydrocephalus, Postdural puncture headache, CSF fistula headache, Headache attributed to spontaneous intracranial hypotension, Headache attributed to neurosarcoidosis, Headache attributed to aseptic (non-infectious) meningitis, Headache attributed to other non-infectious inflammatory disease, Headache attributed to lymphocytic hypophysitis, Syndrome of transient headache, Neurological deficits with cerebrospinal fluid lymphocytosis, Headache attributed to intracranial neoplasm, Headache attributed to carcinomatous meningitis, Headache attributed to hypothalamic or pituitary hyper- or hyposecretion, Headache attributed to intrathecal injection, Headache attributed to epileptic seizure, Headache attributed to chiari malformation type I and Headache attributed to other non-vascular intracranial disorder.

Also described herein and not part of the invention is that the Secondary headache is a headache attributed to a substance or its withdrawal is selected from the group comprising Nitric oxide donor-induced headache, Phosphodiesterase inhibitor-induced headache, Carbon monoxide-induced headache, Alcohol-induced headache, Headache induced by food or additive, Cocaine-induced headache, Histamine-induced headache, Calcitonin gene-related peptide-induced headache, Headache attributed to exogenous acute pressor agent, Headache attributed to occasional use of non-headache medication, Headache attributed to long-term use of non-headache medication, Headache attributed to exogenous hormone, Headache attributed to use of or exposure to other substance, Ergotamine-overuse headache, Triptan overuse headache, Simple analgesic-overuse headache, Opioid-overuse headache, Combination-analgesic-overuse headache, Medication-overuse headache attributed to multiple drug classes not individually overused, Medication-overuse headache attributed to unverified overuse of multiple drug classes, Medication-overuse headache attributed to other medication, Caffeine-withdrawal headache, Opioid-withdrawal headache, Oestrogen-withdrawal headache and Headache attributed to withdrawal from chronic use of other substance.

Also described herein and not part of the invention is that the Secondary headache is a headache attributed to infection is selected from the group comprising Headache attributed to bacterial meningitis or meningoencephalitis, Headache attributed to bacterial meningitis or meningoencephalitis, Headache attributed to viral meningitis or encephalitis, Headache attributed to intracranial fungal or other parasitic infection, Headache attributed to brain abscess, Headache attributed to subdural empyema, Headache attributed to systemic bacterial infection, Headache attributed to systemic viral infection and Headache attributed to other systemic infection.

Also described herein and not part of the invention is that the Secondary headache is a headache attributed to disorder of homoeostasis selected from the group comprising High-altitude headache, Headache attributed to aeroplane travel, Hiving headache, Sleep apnoea headache, Dialysis headache, Headache attributed to phaeochromocytoma, Headache attributed to hypertensive crisis without hypertensive encephalopathy, Headache attributed to hypertensive encephalopathy, Headache attributed to pre-eclampsia or eclampsia, Headache attributed to autonomic dysreflexia, Headache attributed to hypothyroidism, Headache attributed to fasting, cardiac cephalalgia and Headache attributed to other disorder of homoeostasis.

Also described herein and not part of the invention is that the Secondary headache is a headache or facial pain attributed to disorder of the cranium, neck, eyes, ears, nose, sinuses, teeth, mouth or other facial or cervical structure selected from the group comprising Headache attributed to disorder of cranial bone, Cervicogenic headache, Headache attributed to retropharyngeal tendonitis, Headache attributed to craniocervical dystonia, Headache attributed to acute glaucoma, Headache attributed to refractive error, Headache attributed to heterophoria or heterotropia (latent or persistent squint), Headache attributed to ocular inflammatory disorder, Headache attributed to trochleitis, Headache attributed to disorder of the ears, Headache attributed to disorder of the nose or paranasal sinuses, Headache attributed to disorder of the teeth or jaw, Headache attributed to temporomandibular disorder and Head or facial pain attributed to inflammation of the stylohyoid ligament.

Also described herein and not part of the invention is that the Secondary headache is a headache attributed to a psychiatric disorder selected from the group comprising Headache attributed to somatization disorder and Headache attributed to psychotic disorder.

Also described herein and not part of the invention is that the Secondary headache is a painful cranial neuropathy and other facial pains selected from the group comprising Classical trigeminal neuralgia, Painful trigeminal neuropathy attributed to acute herpes zoster, Post-herpetic trigeminal neuropathy, Painful post-traumatic trigeminal neuropathy, Painful trigeminal neuropathy attributed to multiple sclerosis plaque, Painful trigeminal neuropathy attributed to space-occupying lesion, Painful trigeminal neuropathy attributed to other disorder, Glossopharyngeal neuralgia, Nervus intermedius (facial nerve) neuralgia, Occipital neuralgia, Optic neuritis, Headache attributed to ischaemic ocular motor nerve palsy, tolosa-hunt syndrome, Paratrigeminal oculosympathetic (Raeder's) syndrome, Recurrent painful ophthalmoplegic neuropathy, Burning mouth syndrome, Persistent idiopathic facial pain, Central neuropathic pain attributed to multiple sclerosis and Central post-stroke pain.

Also described herein and not part of the invention is that the disease is an autoimmune disease, preferably selected from the group comprising Acute disseminated encephalomyelitis, Addison's disease, Agammaglobulinemia, Alopecia areata, Amyotrophic lateral sclerosis, Ankylosing Spondylitis, Anti-NMDA receptor encephalitis, Antiphospholipid syndrome, Antisynthetase syndrome, Atopic allergy, Atopic dermatitis, Autoimmune aplastic anemia, Autoimmune cardiomyopathy, Autoimmune enteropathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Balo concentric sclerosis, Behçet's disease, Berger's disease, Bickerstaff's encephalitis, Blau syndrome, Bullous pemphigoid, Castleman's disease, Celiac disease, Chagas disease, Chronic inflammatory demyelinating polyneuropathy, Chronic recurrent multifocal osteomyelitis, Chronic obstructive pulmonary disease, Churg-Strauss syndrome, Cicatricial pemphigoid, Cogan syndrome, Cold agglutinin disease, Complement component 2 deficiency, Contact dermatitis, Cranial arteritis, CREST syndrome, Crohn's disease, Cushing's Syndrome, Cutaneous leukocytoclastic angiitis, Dego's disease, Dercum's disease, Dermatitis herpetiformis, Dermatomyositis, Diabetes mellitus type 1, Diffuse cutaneous systemic sclerosis, Dressler's syndrome, Drug-induced lupus, Discoid lupus erythematosus, Eczema, Endometriosis, Enthesitis-related arthritis, Eosinophilic fasciitis, Eosinophilic gastroenteritis, Eosinophilic pneumonia, Epidermolysis bullosa acquisita, Erythema nodosum, Erythroblastosis fetalis, Essential mixed cryoglobulinemia, Evan's syndrome, Fibrodysplasia ossificans progressiva, Fibrosing alveolitis, Gastritis, Gastrointestinal pemphigoid, Glomerulonephritis, Goodpasture's syndrome, Graves' disease, Guillain-Barré syndrome, Hashimoto's encephalopathy, Hashimoto's thyroiditis, Henoch-Schonlein purpura, Herpes gestationis, Hidradenitis suppurativa, Hughes-Stovin syndrome, Hypogammaglobulinemia, Idiopathic inflammatory demyelinating diseases, Idiopathic pulmonary fibrosis, Idiopathic thrombocytopenic purpura, IgA nephropathy, Inclusion body myositis, Chronic inflammatory demyelinating polyneuropathy, Interstitial cystitis, Juvenile rheumatoid arthritis, Kawasaki's disease, Lambert-Eaton myasthenic syndrome, Leukocytoclastic vasculitis, Lichen planus, Lichen sclerosus, Linear IgA disease, Linear IgA disease, Lupus erythematosus, Majeed syndrome, Ménière's disease, Microscopic polyangiitis, Miller-Fisher syndrome, Mixed connective tissue disease, Morphea, Mucha-Habermann disease, Multiple sclerosis, Myasthenia gravis, Microscopic colitis, Myositis, Narcolepsy, Neuromyelitis optica, Occular cicatricial pemphigoid, Opsoclonus myoclonus syndrome, Ord's thyroiditis, Palindromic rheumatism, pediatric autoimmune neuropsychiatric disorders associated with streptococcus, Paraneoplastic cerebellar degeneration, Paroxysmal nocturnal hemoglobinuria, Parry Romberg syndrome, Parsonage-Turner syndrome, Pars planitis, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, Primary sclerosing cholangitis, Progressive inflammatory neuropathy, Psoriasis, Psoriatic arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatoid arthritis, Rheumatic fever, Sarcoidosis, Schizophrenia, Schmidt syndrome, Schnitzler syndrome, Scleritis, Scleroderma, Serum Sickness, Sjögren's syndrome, Spondyloarthropathy, Still's disease, Stiff person syndrome, Subacute bacterial endocarditis, Subacute bacterial endocarditis, Susac's syndrome, Sweet's syndrome, Sympathetic ophthalmia, Takayasu's arteritis, Temporal arteritis, Thrombocytopenia, Tolosa-Hunt syndrome, Transverse myelitis, Ulcerative colitis, Undifferentiated connective tissue disease, Undifferentiated spondyloarthropathy, Urticarial vasculitis, Vasculitis, Vitiligo and Wegener's granulomatosis.

In a preferred embodiment, the term "diagnosis", as used herein, refers to any kind of procedure aiming at obtaining information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a certain treatment, for example the administration of immunosuppressive drugs. In other words, the term "diagnosis" comprises not only diagnosing, but also prognosticating and/or monitoring the course of a disease or disorder.

In many cases the mere detection, in other words determining whether or not detectable levels of the antibody are present in the sample, is sufficient for the diagnosis. If the autoantibody can be detected, this will be information instrumental for the clinician's diagnosis and indicates or indicates an increased likelihood that the patient suffers from a disease. In a preferred embodiment, the relative concentration of the antibody in the serum, compared to the level that may be found in the average healthy subject, may be determined. While in many cases it may be sufficient to determine whether or not autoantibodies are present or detectable in the sample, the method carried out to obtain information instrumental for the diagnosis may involve determining whether the concentration is at least 0.1, preferably 0.2, 0.5, 1, 2, 5, 10, 20, 25, 50, 100, 200, 500, 1000, 10000 or 100000 times higher than the concentration found in the average healthy subject.

The person skilled in the art will appreciate that a clinician does usually not conclude whether or not the patient suffers or is likely to suffer from a disease, condition or disorders solely on the basis of a single diagnostic parameter, but needs to take into account other aspects, for example the presence of other autoantibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred embodiment, the meaning of any symptoms or diseases referred to throughout this application is in line with the person skilled in the art's understanding as of November 20, 2014.

Described herein and not part of the invention is that the term "diagnosis" does preferably not imply that the diagnostic methods or agents will be definitive and sufficient to finalize the diagnosis on the basis of a single test, let alone parameter, but may refer to a contribution to what is referred to as a "differential diagnosis", i. e. a systematic diagnostic procedure considering the likelihood of a range of possible conditions on the basis of a range of diagnostic parameters. Consequently, the method, polypeptide or use, optionally for determining whether a patient suffers from the a disease, may comprise obtaining a sample from a patient, preferably a human patient, determining whether an autoantibody binding to NBC1 is present in said sample, wherein said determining is performed by contacting the sample with the polypeptide and detecting whether binding occurs between said polypeptide and said autoantibody, preferably using a labeled secondary antibody, more preferably using a method from the group comprising radioimmunoassay, Western blot, line blot, ELISA, indirect and immunofluorescence, wherein said autoantibody binds to said polypeptide if present in the sample, and diagnosing the patient as suffering or being more likely to suffer from said neurological disorder or cancer if the autoantibody was determined to be present in the sample.

The term "diagnosis" may also refer to a method or agent used to distinguish between two or more conditions associated with similar or identical symptoms.

The term "diagnosis" may also refer to a method or agent used to choose the most promising treatment regime for a patient. In other words, the method or agent may relate to selecting a treatment regimen for a subject. For example, the detection of autoantibodies may indicate that an immunosuppressive therapy is to be selected, which may include administrating to the patient one or more immunosuppressive drugs.

The present invention relates to a complex comprising an autoantibody, binding to the inventive polypeptide. Such a complex may be used or detected as part of a method for diagnosing a disease. A liquid sample comprising antibodies from a subject may be used to practice the method. Such a liquid sample may be any bodily fluid comprising a representative set of antibodies from the subject, preferably a sample comprising antibodies of the IgG immunglobulin class from the subject, more preferably from the group comprising IgG1, IgG2 and IgG4 subclasses, most preferably from the IgG4 subclass. For example, a sample may be cerebrospinal fluid (CSF), blood or blood serum, lymph, insterstitial fluid and is preferably serum or CSF, more preferably serum.

The step contacting a liquid sample comprising antibodies with the polypeptide may be carried out by incubating an immobilized form of said polypeptide in the presence of the sample comprising antibodies under conditions that are compatible with the formation of the complex comprising said polypeptide and an autoantibody, binding to the polypeptide. The liquid sample, then depleted of antibodies binding to the polypeptide may be removed subsequently, followed by one or more washing steps. Finally the complex comprising the antibody and the polypeptide may be detected. In a preferred embodiment, the term "conditions compatible with the formation of the complex" are conditions that allow for the specific antigen-antibody interactions to build up the complex comprising the polypeptide an the antibody. In a preferred embodiment such conditions may comprise incubating the polypeptide in sample diluted 1:100 in PBS buffer for 30 minutes at 25 °C. In a preferred embodiment, the term "autoantibody", as used herein, refers to an antibody binding specifically to an endogenous molecule of the animal, preferably mammal, which produces said autoantibody, wherein the level of such antibody is more preferably elevated compared the average of any other antibodies binding specifically to such an endogenous molecule. The autoantibody is an autoantibody binding to NBC1. Such an autoantibody may be isolated from samples taken from patients suffering from the neurological disorder characterized by two or more, preferably all symptoms selected from the group comprising mixed movement disorder, acute decline of visual performance, dysarthria and dysphagia.

In a preferred embodiment, the detection of the complex for the prognosis, diagnosis, methods or test kit comprises the use of a method selected from the group comprising immunodiffusion techniques, immunoelectrophoretic techniques, light scattering immunoassays, light scattering immunoassays, agglutination techniques, labeled immunoassays such as those from the group comprising radiolabeled immunoassay, enzyme immunoassays, chemiluminscence immunoassays and immunofluorescence techniques. The person skilled in the art is familiar with these methods, which are also described in the state of the art, for example in Zane, H. D. (2001), Immunology - Theoretical & Practical Concepts in Laboratory Medicine, W. B. Saunders Company, in particular in Chapter 14.

Alternatively, a sample comprising tissue comprising the polypeptide rather than a liquid sample may be used. The tissue sample is preferably from a tissue expressing endogenous NBC1. In a preferred embodiment, the level of NBC1 exceeds the level of NBC1 endogenously expressed under normal conditions. Such a sample, which may be in the form of a tissue section fixed on a carrier, for example a glass slide for microscopic analysis, may then be contacted with the inventive autoantibody, binding to the polypeptide. The antibody is preferably labeled to allow for distinction from endogenous antibodies binding to the polypeptide, so that newly formed complexes may be detected and, optionally, quantified. If the amount of complexes formed is lower than the amount found in a sample taken from a healthy subject, the subject from whom the sample examined has been taken is likely to suffer from a disease.

Any data demonstrating the presence or absence of the complex comprising the antibody and the polypeptide may be correlated with reference data. For example, detection of said complex indicates that the patient who provided the sample analyzed has suffered, is suffering or is likely to suffer in the future from a disease. If a patient has been previously diagnosed and the method for obtaining diagnostically relevant information is run again, the amount of complex detected in both runs may be correlated to find out about the progression of the disease and/or the success of a treatment. For example, if the amount of complex is found to increase, this suggests that the disorder is progressing, likely to manifest in the future and/or that any treatment attempted is unsuccessful.

A microplate, membrane ELISA, dot blot, or line blot may be used to carry out the diagnostic method The person skilled in the art is familiar with the experimental setup, which is described in the state of the art (Raoult, D., and Dasch, G. A. (1989), The line blot: an immunoassay for monoclonal and other antibodies. Its application to the serotyping of gram-negative bacteria. J. Immunol. Methods, 125 (1- 2), 57-65; WO2013041540). Briefly, the one or more antigen of interest, in the case of the present invention the polypeptide, may be attached to a carrier, for example nitrocellulose membrane, often in combination with additional antigens and controls. The nitrocellulose carrier is subsequently exposed to a sample comprising antibodies such as diluted serum. If the sample comprises an antibody binding to the antigen, a complex is formed which may be detected, preferably by incubation with a secondary antibody binding to the constant region of the first antibody, which secondary antibody comprises a detectable label, for example a radioactive isotope, a fluorescent dye or, in a preferred embodiment, an active enzyme fused or linked to the secondary antibody, such as alkaline phosphatase, which may be readily assayed using chromogenic substrates followed by simple visual examination. Suitable reagents, devices and software packages are commercially available, for example from EUROIMMUN, Lübeck, Germany.

The prognosis, diagnosis, methods or test kit may contemplates the use of indirect immunofluorescence. The person skilled in the art is familiar with such techniques and the preparation of suitable samples, which are described in the state of the art (US4647543; Voigt, J., Krause, C., Rohwäder, E, Saschenbrecker, S., Hahn, M., Danckwardt, M., Feirer, C., Ens, K, Fechner, K, Barth, E, Martinetz, T., and Stöcker, W. (2012), Automated Indirect Immunofluorescence Evaluation of Antinuclear Autoantibodies on HEp-2 Cells," Clinical and Developmental Immunology, vol. 2012, doi:10.1155/2012/65105; Bonilla, E., Francis, L., Allam, F., et al., Immuno-fluorescence microscopy is superior to fluorescent beads for detection of antinuclear antibody reactivity in systemic lupus erythematosus patients, Clinical Immunology, vol. 124, no. 1, pp. 18-21, 2007). Briefly, a carrier, such as a cover glass for use in microscopy, is coated with cells or tissue sections comprising the antigen, in the case of the present invention the polypeptide comprising one or more sequences of NBC1 or a variant thereof. The carrier comprising the antigen is exposed to a patient sample comprising antibodies such as diluted serum. If the sample comprises an antibody binding to the antigen, the resulting complex may be detected, preferably by incubation with a secondary antibody comprising a fluorescent dye such as fluorescein, followed by visual examination using fluorescence microscopy. Suitable reagents, devices and software packages are commercially available, for example from EUROIMMUN, Lübeck, Germany.

A sample subjected to a test determining only whether an autoantibody binding to NBC1 is present, but it is preferred that diagnostic methods, tests, devices and the like contemplate determining the presence of autoantibodies against a variety of antigens relating to neurological autoimmune disease or variants thereof, for example Hu, Yo, Ri, CV2, PNMA1, PNMA2, DNER/Tr, ARHGAP26, NBC1, CARPVIII, Zic4, Sox1, MAG, MP0, MBP, GAD65, amphiphysin, recoverin, GABA A receptor, GABA B receptor, glycine receptor, gephyrin, IgLON5, DPPX, aquaporin-4, MOG, NMDA receptor, AMPA receptors, GRM1, GRM5, LGI1 and CASPR2. Therefore, the method, use, kit, device or the like may comprise two or more, preferably three, four, five or more antigens or variants thereof selected from the group comprising Hu, Yo, Ri, CV2, PNMA1, PNMA2, DNER/Tr, ARHGAP26, NBC1, CARPVIII, Zic4, Sox1, MAG, MP0, MBP, GAD65, amphiphysin, recoverin, Homer 3, GABA A receptor, GABA B receptor, glycine receptor, gephyrin, IgLON5, DPPX, aquaporin-4, MOG, NMDA receptor, AMPA receptors, GRM1, CDR2L, GRM5, LGI1, CARPVIII and CASPR2, preferably from the group comprising Yo, CDR2L, DNER/Tr, ARHGAP26, Homer 3 and CARPVIII, more preferably ARHGAP26, Homer 3 and CARPVIII, which antigens are preferably immobilized, for example on a line blot.

According to the teachings of the present invention, an autoantibody binding to the polypeptide used for the diagnosis of a disease is provided. The person skilled in the art is familiar with methods for purifying antibodies, for example those described in Hermanson, G. T., Mallia, A. K., and Smith, P. K. (1992), Immobilized Affinity Ligand Techniques, San Diego: Academic Press. Briefly, an antigen binding specifically to the antibody of interest, which antigen is the polypeptide, is immobilized and used to purify, via affinity chromatography, the antibody of interest from an adequate source. A liquid sample comprising antibodies from a patient suffering from the neurological disorder identified by the inventors may be used as the source.

According to the invention, an autoantibody in complex with a polypeptide comprising NBC1 is provided The term "antibody", as used herein, may refers to any immuglobulin-based binding moieties, more preferably one comprising at least one immunoglobulin heavy chain and one immunoglobulin light chain, including, but not limited to monoclonal and polyclonal antibodies as well as variants of an antibody, in particular fragments, which binding moieties are capable of binding to the respective antigen, more preferably binding specifically to it. In a preferred embodiment, the term "binding specifically", as used herein, means that the binding is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7. The antibody in complex with a polypeptide comprising NBC1 is isolated or Preferably the the autoantibody, may be from the IgG class, most preferably from the group of subclasses comprising IgG1, IgG2 and IgG4, in particular IgG2. In another preferred embodiment, the antibody is a mammalian antibody, more preferably a primate antibody, most preferably a human antibody. The antibody may be glycosylated or non-glycosylated. The person skilled in the art is familiar with methods that may be used for the identification, production and purification of antibodies and variants thereof, for examples those described in EP 2 423 226 A2 and references therein. The antibody in complex with a polypeptide comprising NBC1 may be used as a diagnostic agent,

Also described herein and not part of the invention is a method for isolating an antibody, preferably an autoantibody, binding to the polypeptide, comprising the steps a) contacting a sample comprising the antibody with the polypeptide such that a complex is formed, b) isolating the complex formed in step a), c) dissociating the complex isolated in step b), and d) separating the antibody from the polypeptide. A sample from a patient suffering from the novel neurological disorder identified by the inventors may be used as the source of antibody. Alternatively the antibody may be a recombinant antibody. It is preferred that the polypeptide is immobilized, for example on the matrix of a column suitable for affinity chromatography or on a magnetic bead, since it is straightforward to separate the complex comprising the polypeptide and the antibody in step b) if such is the case. Subsequently, the antibody may be separated from the immobilized antigen in step c), for example by eluting the antibody by addition of an excess of non-immobilized antigen or by adding an agent interfering with intramolecular interactions, for example guanidinium chloride or sodium chloride at a high concentration, the latter if that electrostatic interactions are essential to maintain the complex. One or more washing steps may be included to increase the purity of the complex and the sensitivity and/or specificity of the assay whenever the complex is formed as part of detection or purification methods. The person skilled in the art is familiar with methods to carry out each of these steps. Suitable methods are described in the state of the art, for example in the Handbooks "Affinity chromatography", "Strategies for Protein Purification" and "Antibody Purification" (2009/2010), published by GE Healthcare Life Sciences, and in in Philips, Terry, M., Analytical techniques in immunochemistry, 1992, Marcel Dekker, Inc.

A pharmaceutical composition is described and not part of the invention comprising the polypeptide, which composition is preferably suitable for administration to a subject, preferably a mammalian subject, more preferably to a human. Such a pharmaceutical composition may comprise a pharmaceutically acceptable carrier. The pharmaceutical composition may, for example, be administered orally, parenterally, by inhalation spray, topically, by eyedrops, rectally, nasally, buccally, vaginally or via an implanted reservoir, wherein the term "parentally", as used herein, comprises subcutaneous, intracutaneous, intravenous, intramuscular, intra-articular, intrasynovial, instrasternal, intrathecal, intralesional and intracranial injection or infusion techniques. The pharmaceutical composition may be provided in suitable dosage forms, for example capsules, tablets and aqueous suspensions and solutions, preferably in sterile form. It may be used in a method of treatment of a disease, which method comprises administering an effective amount of the polypeptide to a subject.

Also described herein and not part of the invention is a medical or diagnostic device comprising the inventive autoantibody in complex with a polypeptide comprising NBC1 and/or the polypeptide is provided. Preferably such a medical or diagnostic device comprises the polypeptide in a form that allows contacting it with an aqueous solution, more preferably the liquid human sample, in a straightforward manner. In particular, the polypeptide comprising may be immobilized on the surface of a carrier, which carrier comprises, but is not limited to glass plates or slides, biochips, microtiter plates, beads, for example magnetic beads, chromatography columns, membranes or the like. Exemplary medical devices include line blots, microplates and biochips. In addition to the polypeptide, the medical or diagnostic device may comprise additional polypeptides, for example positive or negative controls or known other antigens binding to autoantibodies of diagnostic value, particularly those related other diseases associated with one or more identical or similar symptoms.

The inventive teachings provide a kit, for diagnosing a disease. Such a kit may comprise instructions detailing how to use the kit and a means for contacting the polypeptide with a bodily fluid sample from a subject, preferably a human subject, for example a line blot, wherein the polypeptide is immobilized on the line blot. Furthermore, the kit may comprise a positive control, for example a batch of autoantibody or recombinant antibody known to bind to the polypeptide and a negative control, for example a protein having no detectable affinity to the polypeptide such as bovine serum albumin. Finally, such a kit may comprise a standard solution of the antibody or antigen for preparing a calibration curve.

The kit comprises a means for detecting an autoantibody binding to the polypeptide, by detecting a complex comprising the polypeptide and an antibody binding to the polypeptide. Such means is an agent that binds to said complex and modifies the complex or carries a label such that makes the complex detectable. Said means is a secondary anti-human antibody binding to the constant region of the autoantibody, preferably a secondary antibody specific for mammalian IgG class antibodies, more preferably mammalian IgG2 class antibodies. A multitude of methods and means for detecting such a complex have been described in the state of the art, for example in Philips, Terry, M., Analytical techniques in immunochemistry, 1992, Marcel Dekker, Inc.

The polypeptide may be provided in the form of a cell comprising and/or expressing a nucleic acid encoding said polypeptide. If a nucleic acid comprising a sequence that encodes for the polypeptide or variant thereof is used, such a nucleic acid may be an unmodified nucleic acid. In a preferred embodiment, the nucleic acid is a nucleic acid that, as such, does not occur in nature and comprises, compared to natural nucleic acid, at least one modification, for example an isotopic content or chemical modifications, for example a methylation, sequence modification, label or the like indicative of synthetic origin. In a preferred embodiment, the nucleic acid is a recombinant nucleic acid or part or a nucleic acid, and is, in a more preferred embodiment, part of a vector, in which it may be functionally linked with a promoter that allows for expression, preferably overexpression of the nucleic acid.

In a preferred embodiment, said nucleic acid is inside a cell capable of expressing it to the effect that the polypeptide or a variant thereof is made and, more preferably, routed to the surface of the cell. Said cell comprising the nucleic acid encoding the polypeptide may be used according to the present invention. The cell may be any kind of cell capable of expressing the nucleic acid, for example a prokaryotic or eukaryotic cell. In preferred embodiment, the cell is a eukaryotic cell such as a yeast cell, a eukaryotic cell from a multicellular organism, for example an insect cell, more preferably a mammalian cell, for example a mouse cell, and most preferably a human cell.

The person skilled in the art is familiar with methods used to synthesize, modify and amplify such a nucleic acid and to transfect cells using such a nucleic acid, preferably in a vector that allows for the transient or permanent maintenance or expression of the nucleic acid in the cell. The person skilled in the art is also familiar with a variety of suitable vectors, of which are commercially available, for example from Origene. For example, a vector encoding for fusion constructs with a C-terminal GFP may be used. The cell may be of eukaryotic or prokaryotic origin and is preferably a mammalian cell, for example a HEK293, CHO or COS-7 cell. The cell comprising the nucleic acid encoding for the polypeptide may be a recombinant cell or an isolated cell wherein the term "isolated" means that the cell is enriched such that, compared to the environment of the wild type of said cell, fewer cells of other differentiation or species or in fact no such other cells are present.

Also described herein and not part of the invention is a method for preventing or treating a disease, comprising the steps a) reducing the concentration of autoantibodies binding to the polypeptide in the subject's blood and/or b) administering one or more immunosuppressive pharmaceutical substances, preferably selected from the group comprising rituximab, prednisone, methylprednisolone, cyclophosphamide, mycophenolatemofetil, intravenous immunoglobulin, tacrolimus, cyclosporine, methotrexate, azathioprine and/or the pharmaceutical composition.

The present invention is further illustrated by the following figures and non-limiting sequences and examples from which further features, embodiments, aspects and advantages of the present invention may be taken.

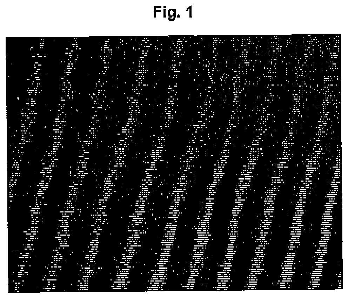

**Fig. 1** shows the distinct IgG staining of primate cerebellum using patient serum.

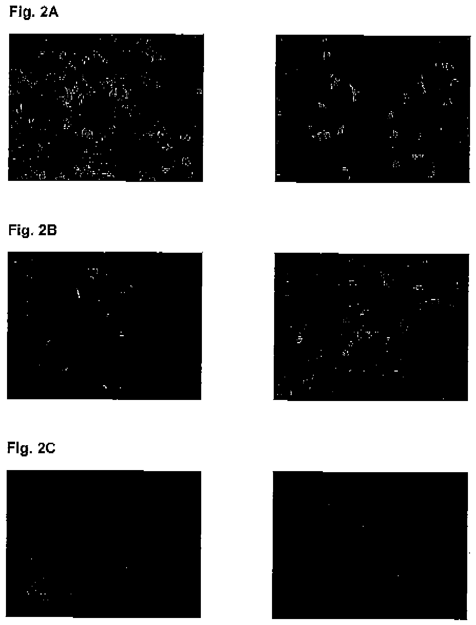

**Fig. 2** shows the results of immunofluorescence analyses of various samples using patient serum as the antibody source. Acetone-fixed (left column) and formalin-fixed samples (right column) were analyzed, more specifically HEK293 cells infected using murine NBC1 isoform 1 (**Fig. 2A**), human NBC1 isoform 3 (**Fig. 2B**) and mock-transfected HECK293 cells (**Fig. 2C**). Whilst HEK293 cells infected with NBC1 show distinct immunofluorescence staining, there virtually no staining if mock-transfected HEK293 cells are used.

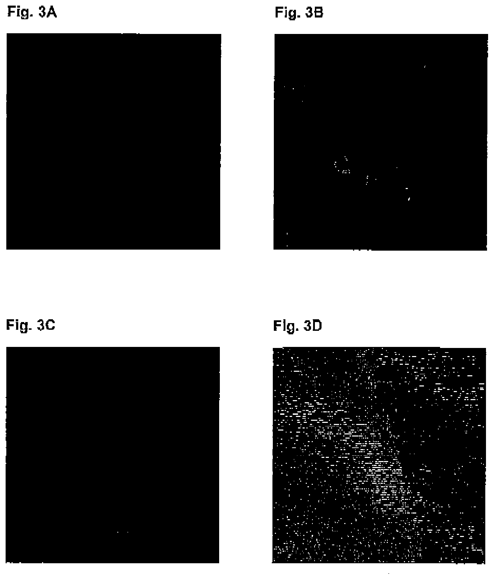

**Fig. 3A** shows the competitive abolition of binding of anti-NBC1 from patient serum to HEK293 cells expressing murine NBC1 in the presence (**Fig. 3A**) and absence (**Fig. 3B**) of lysate made of HEK293 expressing murine NBC1. In the experiments shown in Figs. **3C** and **3D**, primate cerebellum was used rather than HEK293 cells in the presence and absence of lysate made of HEK293 expressing murine NBC1, respectively.

### Examples

The following examples illustrate that autoantibodies to NBC1 are present in samples from patients having neurological symptoms, whilst no such antibodies could be detected in samples from healthy subjects. These autoantibodies could be identified on account of their ability to produce distinct immunofluorescence staining patterns on mammalian tissues. They recognize various mammalian forms of NCB1, including primate, human, mouse and rat.

### Initial serological work-up

Indirect immunofluorescence assay (IFA) was performed according to the standard incubation protocol provided by EUROIMMUN. Briefly, slides with 4 µm cryosections of mammalian brain tissues were incubated with the patient serum diluted 1:10 to 1:10000 in PBS, 0.5% Tween-20 (sample buffer) for 30 minutes at 25 °C. After thorough washing with sample buffer the slide was incubated with anti-human-IgG conjugated to fluorescein-isothiocyanate (FITC, EUROIMMUN) for 30 minutes at 25 °C. After another washing step the slides were inspected with a fluorescence microscope.

In total, 465 samples from patients suffering from a variety of neurological symptoms showed IgG reactivity against mammalian neural tissue cryosections in indirect immunofluorescence. Further monospecific analyses were conducted with recombinant HEK293 cells expressing 32 different neural autoantigens: Hu, Yo, Ri, CV2, SOX1, PNMA1, PNMA2, ARHGAP26, NBC1, CARP, Homer 3, ZIC4, DNER/Tr, GAD65, GAD67, amphiphysin, recoverin, GABA A receptor, GABA B receptor, IgLON5, glycine receptor, DPPX, glutamate receptors (types NMDA, AMPA, mGluR1, mGluR5), LGI1, CASPR2, AQP4 (M1 and M23), MOG, ATP1A3. None revealed any specific reactivity.

A subgroup of 225 sera showed a common homogenous IgG staining of both granular and molecular layer, but no staining of Purkinje cells was observed (**Fig. 1**).

*Identification of electrogenic sodium bicarbonate cotransporter* as *the target autoantigen* Histo-immunoprecipitation was performed using one of the patient sera (index sample, titer: 1:320) in 1:100 dilution together with cerebellum cryosections of rat or pig. Alternatively, samples from healthy blood donors were used as controls. Immunoprecipitates contained high amounts of IgG when patient serum was used whereas they were generally low after incubation of sera from healthy controls. Next to the immunoglobulins the immunoprecipitated patient serum showed protein bands corresponding to molecular masses of approximately 120 kDa in SDS-PAGE stained with colloidal coomassie. The band was absent in the control samples. The 120 kDa protein precipitated from rat tissue was identified as electrogenic sodium bicarbonate cotransporter from *Rattus norvegicus*

Western Blot analysis with a monoclonal NBC1 antibody showed a strong reaction at 120 kDa of the immunoprecipitate obtained with the patient serum, while there were no reactions with fractions obtained with control

As a proof for the correct antigen identification, the reference patient sample and the samples from healthy controls were then analyzed by IFA with HEK293 expressing either murine NBC1 IF1 (UNIPROT acc. # 088343) or human NBC1 IF3 (Q9Y6R1-1) and with mock-transfected control cells (**Fig. 2**). The reference sample produced a characteristic staining pattern on cells expressing NBC1 independent of the type of fixation (acetone or 1% (w/v) formalin). In contrast, control sera did not show any reactivity.

The congruence of the reference patient autoantibodies' target and NBC1 was further demonstrated by the dose-dependent competitive abolition of antibody binding to brain tissue by HEK293 fractions containing NBC1 (**Fig. 3**). Antibody binding was unaffected when comparable fractions from mock-transfected cells were used.

## Claims

1. A test kit for the *in vitro* diagnosis of a disease associated with neurological symptoms, comprising a polypeptide comprising NBC1 or a variant thereof having a sequence identity of at least 80 % to the sequence of NBC1 as represented by the UNIPROT accession number Q9Y6R1,
wherein the variant has biological activity,
wherein the test kit comprises, in addition, a means for detecting the complex comprising an autoantibody binding to NBC1,
and wherein said means is a detectably labeled secondary antibody binding to the constant region of human IgG class antibodies.

2. The test kit according to claim 1, wherein the polypeptide is provided in the form of a cell comprising a nucleic acid encoding said polypeptide or in the form of a tissue comprising said polypeptide.

3. The test kit according to claim 1, wherein the polypeptide is a recombinant and/or isolated polypeptide.

4. An isolated human autoantibody binding to a polypeptide comprising NBC1, wherein the autoantibody is in complex with a polypeptide comprising NBC1.

## Patentansprüche

1. Testkit für die in-vitro-Diagnose einer mit neurologischen Symptomen assoziierten Krankheit, umfassend ein Polypeptid umfassend NBC1 oder eine Variante davon, die eine Sequenzidentität von wenigstens 80 % zu der Sequenz von NBC1 aufweist, wie sie durch die UNIPROT-Zugangsnummer Q9Y6R1 wiedergegeben ist,
wobei die Variante biologische Aktivität aufweist,
wobei das Testkit zusätzlich ein Mittel zum Nachweisen des Komplexes umfassend einen an NBC1 bindenden Autoantikörper umfasst,
und wobei das genannte Mittel ein nachweisbar markierter Sekundärantikörper ist, der an die konstante Region von humanen Antikörpern der IgG-Klasse bindet.

2. Testkit nach Anspruch 1, wobei das Polypeptid in Form einer Zelle umfassend eine Nukleinsäure, die für das genannte Polypeptid kodiert, oder in Form eines Gewebes umfassend das genannte Polypeptid bereitgestellt ist.

3. Testkit nach Anspruch 1, wobei das Polypeptid ein rekombinantes und/oder isoliertes Polypeptid ist.

4. Isolierter humaner Autoantikörper, der an ein Polypeptid umfassend NBC1 bindet, wobei der Autoantikörper in einem Komplex mit einem Polypeptid umfassend NBC1 vorliegt.

## Revendications

1. Kit d'essai pour le diagnostic *in vitro* d'une maladie associée à des symptômes neurologiques, comprenant un polypeptide comprenant NBC1 ou un variant de celui-ci ayant une identité de séquence d'au moins 80 % avec la séquence de NBC1 telle que représentée par le numéro d'accession UNIPROT Q9Y6R1,
le variant ayant une activité biologique,
le kit d'essai comprenant en outre un moyen pour détecter le complexe comprenant un anticorps se liant à NBC1,
et ledit moyen étant un anticorps secondaire marqué d'une manière détectable se liant à la région constante des anticorps de la classe des IgG humains.

2. Kit d'essai selon la revendication 1, dans lequel le polypeptide est fourni sous forme d'une cellule comprenant un acide nucléique codant pour ledit polypeptide ou sous la forme d'un tissu comprenant ledit polypeptide.

3. Kit d'essai selon la revendication 1, dans lequel le polypeptide est un polypeptide recombinant et/ou isolé.

4. Autoanticorps humain isolé se liant à un polypeptide comprenant NBC1, l'autoanticorps étant un complexe avec un polypeptide comprenant NBC1.
